(19) Europäisches Patentamt
European Patent Office
Office européen des brevets

(11) Veröffentlichungsnummer : **0 051 859**
**B1**

(12) # EUROPÄISCHE PATENTSCHRIFT

(45) Veröffentlichungstag der Patentschrift : **25.07.84**

(51) Int. Cl.³ : **C 07 C 31/08**, C 07 C 31/10, C 07 C 29/00, B 01 J 31/18, B 01 J 31/24, B 01 J 31/30

(21) Anmeldenummer : **81109524.9**

(22) Anmeldetag : **05.11.81**

(54) **Verfahren zur Herstellung von Ethanol aus Methanol.**

(30) Priorität : **11.11.80 DE 3042434**

(43) Veröffentlichungstag der Anmeldung : **19.05.82 Patentblatt 82/20**

(45) Bekanntmachung des Hinweises auf die Patenterteilung : **25.07.84 Patentblatt 84/30**

(84) Benannte Vertragsstaaten : **BE CH DE FR GB IT LI NL SE**

(56) Entgegenhaltungen :
**EP-A- 0 010 373**
**GB-A- 979 908**
**US-A- 4 233 466**
**US-A- 4 239 924**

(73) Patentinhaber : **Ruhrchemie Aktiengesellschaft**
**Bruchstrasse 219**
**D-4200 Oberhausen 13 (DE)**

(72) Erfinder : **Cornils, Boy, Dr. Dipl.-Chem.**
**Friedrich-Ebert-Strasse 45**
**D-4220 Dinslaken (DE)**
Erfinder : **Frohning, Carl Dieter, Dr. Dipl.-Chem.**
**Elsenbruch 1**
**D-4200 Oberhausen 13 (DE)**
Erfinder : **Diekhaus, Gerhard, Dr. Dipl.-Chem.**
**Walsumermarkstrasse 89**
**D-4200 Oberhausen 14 (US)**
Erfinder : **Wiebus, Ernst**
**Ferdinandstrasse 77**
**D-4200 Oberhausen 14 (DE)**
Erfinder : **Bahrmann, Helmut, Dr. Dipl.-Chem.**
**Im Freihof 56**
**D-4224 Hünxe (DE)**

(74) Vertreter : **Reichelt, Karl-Heinz, Dr.**
**m. Br. Ruhrchemie Aktiengesellschaft Abt. PLD Postfach 13 01 60**
**D-4200 Oberhausen 13 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 051 859 B1

Jouve, 18, rue St-Denis, 75001 Paris, France

**Beschreibung**

Die Erfindung betrifft ein Verfahren zur Herstellung von Ethanol aus Methanol und Synthesegas, d. h. dem Gemisch von Kohlenmonoxid und Wasserstoff in Gegenwart von Kobalt und Ruthenium als Katalysatoren. Diese Reaktion, sie wird als Homologisierung bezeichnet, ermöglicht es, ausgehend von Methanol, durch Einführung einer oder mehrerer $CH_2$-Gruppen höhere homologe Alkohole herzustellen.

Die Homologisierung findet im verstärkten Maße Interesse, da sie einen Weg zur Gewinnung höherer Alkohole eröffnet, der unabhängig von der Verwendung von Erdöl ist. Als Einsatzstoff wird Synthesegas bzw. daraus hergestelltes Methanol benötigt; Synthesegas ist, z. B. aus Kohle oder Erdgas nach verschiedenen, technisch bewährten Prozessen zugänglich.

Es ist seit langem bekannt (vgl. z. B. DE-PS 867 849), Methanol mit Wasserstoff und Kohlenmonoxid in Gegenwart eines wasserlöslichen Kobaltkatalysators bei hohen Temperaturen und Drücken in Ethanol umzuwandeln. Die Reaktion verläuft nach folgender Summengleichung:

$$CH_3OH + CO + 2\,H_2 \longrightarrow C_2H_5OH + H_2O$$

wobei in untergeordnetem Maße höhere Alkohole entsprechend

$$CH_3OH + n(CO + 2\,H_2) \longrightarrow CH_3(CH_2)_nOH + n\,H_2O$$

gebildet werden können.

Während ursprünglich für die Reaktion ausschließlich Kobalt als Katalysator verwendet wurde, gewannen im Laufe der Zeit Mehrkomponenten-Katalysatoren zunehmend Bedeutung.

In der US-PS 32 85 948 ist die Herstellung von Ethanol aus Methanol unter Verwendung von Kobalt als Katalysator, Jod oder einer Jodverbindung als erstem und einem Rutheniumhalogenid oder einem Osmiumchlorid als weiterem Promotor beschrieben. Die beanspruchten Maßnahmen sollen zu einer Erhöhung der Selektivität der Reaktion zu Ethanol führen.

Das gleiche Ziel wird nach dem Verfahren der DE-OS 26 25 627 mit einem Katalysatorsystem angestrebt, das aus Kobalt, einem Halogenid als Promotor und einem tertiären Phosphin besteht; die Umsetzung erfolgt in einem Kohlenwasserstoff als Lösungsmittel.

Nach der Lehre der US-PS 41 33 966 gewinnt man Ethanol aus Methanol und Synthesegas unter Verwendung eines aus Kobaltacetylacetonat, einer organischen Verbindung eines Elementes der Gruppe VA des Periodensystems der Elemente, einer Rutheniumverbindung und einer Jodverbindung bestehenden Katalysators.

Aus der EP-A1-00 10 373 ist ein Verfahren zur Herstellung von Ethanol und/oder Acetaldehyd durch Umsetzung von Methanol mit Synthesegas bekannt, das in Gegenwart eines Katalysators aus Kobalt, Jodid oder Bromid und einem mehrzähnigen Liganden mit Stickstoff-, Phosphor-, Arsen-, Wismut- oder Antimonatomen durchgeführt wird. Die Reaktion kann auch in der Weise erfolgen, daß zusätzlich Ruthenium zugegen ist.

Trotz der vorstehend beschriebenen Maßnahmen reicht die erzielte Selektivität der Reaktion für eine technische Anwendung nicht aus. Hierbei ist zu berücksichtigen, daß die Nebenprodukte nicht nur in großer Menge, sondern in Form zahlreicher, unterschiedlicher Einzelverbindungen anfallen. So werden neben den erwünschten Alkoholen z. B. Methan, Ethan, Propan, verschiedene Ether sowie Methylacetat, Ethylacetat, Propylacetat, Acetaldehyd-dimethylacetal, Acetaldehyd-methylethylacetal und Acetaldehyddiethylacetal gebildet. Bei industrieller Nutzung des Prozesses ist daher ein hoher Aufwand erforderlich, um die aus den Nebenprodukten gewinnbaren Wertproduktanteile, z. B. durch Hydrierung, Verseifung und Destillation, zu isolieren.

Eine Verbesserung der Selektivität der Umsetzung durch Zusatz eines Lösungsmittels zu den Reaktionspartnern hat eine erhebliche Abnahme des Umsatzes bezogen auf Reaktorvolumen und Zeit zur Folge.

Nach den bekannten Verfahren kann man also entweder Methanol mit zufriedenstellender Selektivität in höhere Alkohole überführen, erzielt dann aber nur geringe Umsätze oder man erreicht hohe Umsätze bei niedriger Selektivität.

Es bestand daher die Aufgabe, eine Arbeitsweise zu entwickeln, die es erlaubt, Methanol in Ethanol mit hoher Selektivität une hohem Umsatz zu überführen, sowie die Anzahl der Nebenprodukte wesentlich zu reduzieren, so daß das Reaktionsgemisch in einfacher Weise aufgetrennt werden kann.

Die Erfindung löst diese Aufgabe mittels eines Verfahrens zur Herstellung von Ethanol durch Umsetzung von Methanol mit Kohlenmonoxid und Wasserstoff bei Drücken von 200 bis 600 bar und Temperaturen von 150 bis 250 °C in Gegenwart von 5 bis 25 Gew.% Wasser, bezogen auf Methanol, und in Gegenwart eines Katalysators, der eine Kobaltverbindung, eine Rutheniumverbindung, Jod oder Jodid und eine zweizähnige organische Phosphorverbindung enthält. Es ist dadurch gekennzeichnet, daß die zweizähnige organische Phosphorverbindung der allgemeinen Formel

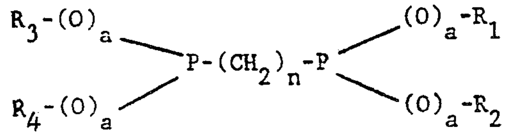

entspricht, wobei a 0 oder 1 ist, n die Zahl 1, 2, 3, 4, 5 oder 6 bedeutet und für den Fall a = 0 $R_1$ und $R_3$ Wasserstoff und $R_2$ und $R_4$ gleiche oder verschiedene, geradkettige oder verzweigte Alkylreste mit 1 bis 16 Kohlenstoffatomen oder gleiche oder verschiedene Arylreste mit 6 bis 15 Kohlenstoffatomen bedeuten und für den Fall daß a = 1 ist, $R_1$, $R_2$, $R_3$ und $R_4$ gleich oder verschieden sind und für Wasserstoff, geradkettige oder verzweigte Alkylreste mit 1 bis 16 Kohlenstoffatomen oder Arylreste mit 6 bis 15 Kohlenstoffatomen bedeuten.

Unter zweizähnigen organischen Phosphorverbindungen werden solche Verbindungen verstanden, die zwei gleichzeitig als Donoren wirkende Phosphoratome enthalten.

Beispiele für Alkyl- und Arylreste in der obigen allgemeinen Formel sind Methyl, Ethyl, Propyl, Butyl, Pentyl, i-Propyl, i-Hexadecyl, Neopentyl, Cyclohexyl, Dicyclopentyl, Phenyl, Tolyl, Naphthyl, Phthalyl.

Die Reste $R_1$ und $R_2$ bzw. $R_3$ und $R_4$ können auch miteinander verbunden sein und auf diese Weise einen Phosphor enthaltenden heterocyclischen Ring bilden.

Die Reste $R_1$ und $R_4$ können über Sauerstoffatome mit dem Phosphoratom verbunden sein. Das gleiche gilt entsprechend der Bedeutung von a für die Verknüpfung beider Phosphoratome über den Alkylrest. Beispiele für Verbindungen entsprechend den beschriebenen Formeln, die im Rahmen der erfindungsgemäßen Arbeitsweise eingesetzt werden, sind 1,3-Bis-(monophenylphosphino)-propan (1), 1,3-Bis-(diphenylphosphino)-propan (2), Bis-(monophenylphosphino)-methan (3), Bis-(diphenyl-phosphino)-methan (4),1,3-Bis-[1,3,2-dioxaphospholanyl-(2)]-propan (5), Bis-[1,3,2-dioxaphospholanyl-(2)]-methan (6), 1,3-Bis-(diphenoxyphosphino)-propan (7), bevorzugt finden 1,3-Bis-(diphenylphosphino)-propan (2), Bis-(diphenylphosphino)methan (4) Anwendung.

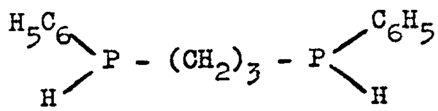 (1)

$(H_5C_6)_2\ P-CH_2-CH_2-CH_2-P(C_6H_5)_2$ (2)

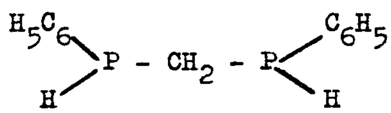 (3)

$(H_5C_6)_2P - CH_2 - P(C_6H_5)_2$ (4)

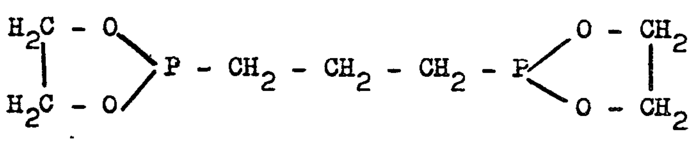 (5)

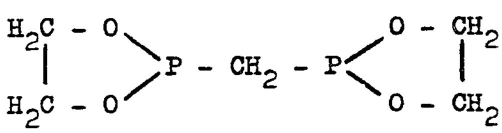 (6)

$(H_5C_6-O)_2-P-CH_2-CH_2-CH_2-P(-O-C_6H_5)_2$ (7)

Die vorstehend beschriebenen Phosphorverbindungen bilden mit den Kobalt- und Rutheniumverbindungen unter den Reaktionsbedingungen Komplexverbindungen, die außerdem Kohlenmonoxid und Wasserstoff enthalten können. Diese Verbindungen stellen einen Bestandteil des wirksamen Katalysatorsystems dar.

Kobalt wird dem Reaktionsgemisch im allgemeinen in Form eines Salzes wie Kobalt-2-ethylhexanoat, Kobaltacetylacetonat, Kobalthalogenid, Kobaltnitrat, als Oxid oder Hydroxid zugesetzt. Besonders bewährt hat sich Kobaltcarbonat. Es ist aber auch möglich, metallisches Kobalt in feinverteilter Form zu verwenden.

Entscheidend ist, daß Kobalt bzw. die Kobaltverbindung mit Kohlenmonoxid und Wasserstoff unter Bildung eines Kobalt-Carbonyls oder -Hydrocarbonyls reagiert.

Ein weiterer Bestandteil des Katalysatorsystems entsprechend dem neuen Verfahren ist Ruthenium, das dem Reaktionsgemisch als Verbindung, die sich unter den Reaktionsbedingungen in Carbonylkomplexe umsetzt, z. B. Rutheniumhalogenid, Ruthenium-2-äthylhexanoat, Ruthenium-acetylacetonat, $(NH_4)_4[Ru_2OCl_{10}]$, vorzugsweise als Chlorid $RuCl_3$ zugesetzt wird.

Schließlich enthält das Katalysatorsystem noch Jod in molekularer oder in ionischer Form. Als Salze des Jods können Alkalimetallsalze und besonders vorteilhaft Kobaltjodid verwendet werden.

Das im erfindungsgemäßen Verfahren eingesetzte Katalysatorsystem kann man dem Reaktionsgemisch in Form seiner einzelnen Bestandteile zuführen. Eine Präformierung der Metallkomplexverbindungen, die Komponenten des Katalysatorsystems sind, ist nicht erforderlich. Das Katalysatorsystem ist wiederholt einsetzbar.

Die Carbonyle bildenden Katalysatoren bzw. Katalysatorkomponenten können in einem hochsiedenden Lösungsmittel wie Diethylenglykolether (Diglyme), Tetraethylenglykolether (Tetraglyme), Neopentylglykol (2,2-Dimethyl-1,3-propandiol), Ethylenglykol oder 2-Ethylhexanol suspendiert bzw. aufgelöst werden. Besonders geeignet als Lösungs- bzw. Suspensionsmittel sind die höher als Ethanol bzw. n-Propanol siedenden organischen Nebenprodukte der Reaktion.

Das als Ausgangsstoff eingesetzte Methanol kann in Form des in technischen Anlagen hergestellten Produktes mit einem Wassergehalt von 4-6 % verwendet werden. Eine zusätzliche Reinigung ist nicht erforderlich.

Das ursprünglich eingesetzte Reaktionsgemisch enthält 5 bis 25 Gew.-% Wasser, bezogen auf Methanol. Durch den Wasserzusatz wird eine Erhöhung des Umsatzes bewirkt. Höhere Wassermengen beeinflussen den Umsatz nur unwesentlich, geringere Mengen haben keine oder nur unerhebliche Umsatzsteigerungen zur Folge. Zweckmäßig wird das Wasser zusammen mit dem Methanol dem Reaktor zugeführt.

Das molare Verhältnis von Kobalt zu Methanol beträgt in der Regel 1 : 20 bis 1 : 10 000 insbesondere 1 : 30 bis 1 : 2 000. Kobalt und Phosphorverbindung werden in der Regel in einem molaren Verhältnis von 1 : 1 bis 1 : 20 vorzugsweise 1 : 1 bis 1 : 5 eingesetzt.

Das Atomverhältnis von Kobalt zu Ruthenium beträgt in der Regel 1 : 0,000 5 bis 1 : 0,5 vorzugsweise 1 : 0,05 bis 1 : 0,1.

Kobalt und Jod werden in der Regel im molaren Verhältnis von 1 : 0,02 bis 1 : 2 insbesondere 1 : 0,1 bis 1 : 1 angewandt.

Das Kohlenmonoxid-/Wasserstoff-Gemisch soll keine die die Aktivität des Katalysatorsystems beeinflussenden Verunreinigungen wie Schwefel enthalten. Kohlendioxid und/oder Stickstoff bis zu 5 Vol.% bezogen auf das Gesamtgemisch schaden nicht.

Der neue Prozeß kann sowohl diskontinuierlich als auch kontinuierlich durchgeführt werden. Im allgemeinen erfolgt die Umsetzung von Methanol, Kohlenmonoxid und Wasserstoff bei Temperaturen von 150 bis 250 °C insbesondere 160 bis 230 °C. Der Druck wird auf Werte zwischen 200 bis 600 bar vorzugsweise 450 bis 600 bar gehalten. Das molare Verhältnis von Wasserstoff zu Kohlenmonoxid im Synthesegas beträgt 1 : 1 bis 10 : 1.

Die nachstehend beschriebenen Beispiele erläutern die Erfindung.

## Beispiel 1

In einem Stahlautoklaven (1 l Inhalt), versehen mit Hubrührer, Temperaturmessung, Probenahmestutzen und einem Gasometer, das zum Auffangen gasförmiger Bestandteile dient, werden 6,25 mol (200 g) Methanol, 1,1 mol (19,80 g) Wasser, 17 mmol (2,02 g $CoCO_3$) Co, 6,7 mmol (1,00 g NaJ) Jodid, 22,1 mmol (9,11 g) 1,3-Bis-(diphenylphosphino)-propan und 1 mmol (0,26 g $RuCl_3 \cdot 3H_2O$) Ru vorgelegt. Anschließend wird mit Synthesegas (CO : $H_2$ = 1 : 3) ein Druck von 550 bar eingestellt, auf 185 °C erhitzt und die Reaktion 6 Stunden lang unter Nachpressen von Synthesegas fortgesetzt.

Nach Abkühlen des Reaktionsgemisches und Entspannen in das Gasometer wird eine Durchschnittsprobe genommen, deren gaschromatographische Zusammensetzung in der Tabelle angegeben ist.

## Beispiel 2

In den Stahlautoklaven der Apparatur gemäß Beispiel 1 werden 4,5 mol (144 g) Methanol, 0,8 mol (14,40 g) Wasser, 134 mmol (15,95 g $CoCO_3$) Co, 53 mmol (7,95 g NaJ) Jodid, 146 mmol (60,15 g) 1,3-Bis-(diphenylphosphino)-propan und 7,90 mmol (3,15 g Ru-III-acetylacetonat) Ru vorgelegt. Anschließend wird mit Synthesegas ($CO : H_2 = 1 : 3$) ein Druck von 280 bar eingestellt, auf 185 °C erhitzt und die Reaktion 3 Stunden lang unter Nachpressen von Synthesegas fortgesetzt.

Die Probenahme erfolgt wie in Beispiel 1 angegeben, die gaschromatographische Zusammensetzung der untersuchten Probe ist in der Tabelle angegeben.

Beispiel 3

Die in Beispiel 2 angefallene Reaktionsmischung wird destillativ von allen flüchtigen Bestandteilen befreit und der trockene Rückstand erneut in die Reaktion, wie in Beispiel 2 angegeben, anstelle des Katalysatorsystems mit 4,5 mol (144,0 g) Methanol und 0,8 mol (14,4 g) Wasser eingesetzt. Anschließend wird mit Synthesegas ($CO : H_2 = 1 : 3$) ein Druck von 550 bar eingestellt, auf 185 °C erhitzt und die Reaktion 3 Stunden lang unter Nachpressen von Synthesegas fortgesetzt.

Die Probenahme erfolgt wie in Beispiel 1 angegeben ist, die gaschromatographische Zusammensetzung der untersuchten Probe ist in der Tabelle angegeben.

Beispiel 4

Es werden die gleichen Bedingungen wie in Beispiel 1 angegeben eingehalten, jedoch wird anstelle von 1,3-Bis-(diphenylphosphino)-propan 22,1 mmol (8,49 g) Bis-(diphenylphosphino)-methan eingesetzt. Die Reaktion wird wie in Beispiel 1 angegeben, durchgeführt.

Beispiel 5

Die in Beispiel 4 angefallene Reaktionsmischung wird destillativ von allen flüchtigen Bestandteilen befreit und der trockene Rückstand erneut in die Reaktion, wie in Beispiel 4 angegeben mit 6,25 mol (200,0 g) Methanol und 1,1 mol (19,80 g) Wasser eingesetzt. Es werden die gleichen Bedingungen wie in Beispiel 1 angegeben eingehalten.

Beispiel 6

In den Stahlautoklaven der Apparatur gemäß Beispiel 1 werden 9,375 mol (300,0 g) Methanol, 1,667 mol (30,0 g) Wasser, 25,50 mmol (3,03 g) $CoCO_3$) Co, 10 mmol (1,50 g NaJ) Jodid, 1,50 mmol (0,60 g Ru-III-acetylacetonat) Ru und 28,10 mmol (7,30 g) 1,3-Bis-(monophenylphosphino)-propan vorgelegt und wie in Beispiel 1 angegeben, weitergearbeitet.

Beispiel 7

Es werden die gleichen Bedingungen wie in Beispiel 6 angegeben eingehalten, jedoch wird anstelle von 1,3-Bis-(mono-phenylphosphino)-propan 28,10 mmol (6,30 g) 1,3-Bis-[1,3,2-dioxaphospholanyl-(2)]-propan eingesetzt. Die Reaktion wird wie in Beispiel 1 angegeben, durchgeführt.

Die Ergebnisse sind den folgenden Tabellen 1 und 2 zu entnehmen.

Tabelle 1

GLC-Analyse (Gew.-%)

| Komponente | Beispiele | | | | | | |
|---|---|---|---|---|---|---|---|
| | 1 | 2 | 3 | 4 | 5 | 6 | 7 |
| Alkane | 3,3 | 2,6 | 6,4 | 3,1 | - | 2,5 | 2,2 |
| Ether | 3,6 | 0,8 | 2,3 | 2,6 | 0,8 | 2,1 | 2,9 |
| Ester | 1,7 | 0,5 | 2,2 | 1,2 | 0,8 | 1,3 | 1,5 |
| Acetale | - | - | - | 1,5 | 1,2 | 0,3 | 0,6 |

(suite)

| Komponente | Beispiele 1 | 2 | 3 | 4 | 5 | 6 | 7 |
|---|---|---|---|---|---|---|---|
| Methanol | 47,7 | 59,5 | 27,7 | 56,0 | 70,7 | 64,0 | 57,6 |
| Ethanol | 40,7 | 29,6 | 53,5 | 31,2 | 24,3 | 27,6 | 32,3 |
| n-Propanol | 2,3 | 5,6 | 5,3 | 1,6 | 0,8 | 0,9 | 1,3 |
| höhere Alkohole | 0,7 | 0,3 | 0,7 | 1,9 | 0,5 | 0,6 | 1,1 |
| Nachlauf | Spur | 1,1 | 1,9 | 0,9 | 0,9 | 0,7 | 0,5 |

Tabelle 2

| | Beispiele 1 | 2 | 3 | 4 | 5 | 6 | 7 |
|---|---|---|---|---|---|---|---|
| Umsatz bezogen auf Methanol (in %) | 52,3 | 40,5 | 72,3 | 44,0 | 29,3 | 36,0 | 42,4 |
| Selektivität zu Ethanol (in %) | 78,0 | 73,1 | 73,9 | 71,0 | 83,8 | 77,0 | 76,0 |
| Selektivität zu Propanol (in %) | 4,0 | 13,8 | 7,4 | 8,0 | 2,8 | 3,0 | 3,0 |

**Ansprüche**

1. Verfahren zur Herstellung von Ethanol durch Umsetzung von Methanol mit Kohlenmonoxid und Wasserstoff bei Drücken von 200 bis 600 bar une Temperaturen von 150 bis 250 °C in Gegenwart von 5 bis 25 Gew.% Wasser, bezogen auf Methanol, und in Gegenwart eines Katalysators, der eine Kobaltverbindung, eine Rutheniumverbindung, Jod oder Jodid und eine zweizähnige organische Phosphorverbindung enthält, dadurch gekennzeichnet, daß die zweizähnige organische Phosphorverbindung der allgemeinen Formel

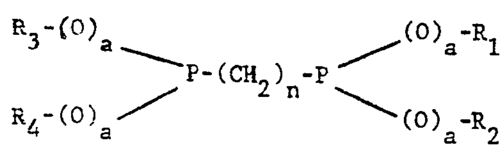

entspricht, wobei a 0 oder 1 ist, n die Zahl 1, 2, 3, 4, 5 oder 6 bedeutet und für den Fall a = 0 $R_1$ und $R_3$ Wasserstoff une $R_2$ und $R_4$ gleiche oder verschiedene, geradkettige oder verzweigte Alkylreste mit 1 bis 16 Kohlenstoffatomen oder gleiche oder verschiedene Arylreste mit 6 bis 15 Kohlenstoffatomen bedeuten und für den Fall daß a = 1 ist, $R_1$, $R_2$, $R_3$ und $R_4$ gleich oder verschieden sind und für Wasserstoff, geradkettige oder verzweigte Alkylreste mit 1 bis 16 Kohlenstoffatomen oder Arylreste mit 6 bis 15 Kohlenstoffatomen bedeuten.

2. Verfahren nach Anspruch 1, dadurch gekennzeichnet, daß für den Fall a = 1 die Reste $R_1$ und $R_2$ bzw. $R_3$ und $R_4$ miteinander verbunden sind.

3. Verfahren nach Anspruch 1 und 2, dadurch gekennzeichnet, daß das molare Verhältnis von Kobalt zu Methanol 1 : 20 bis 1 : 10 000 vorzugsweise 1 : 30 bis 1 : 2 000 und das molare Verhältnis von Kobalt zu Phosphorverbindung 1 : 1 bis 1 : 20 vorzugweise 1 : 1 bis 1 : 5 beträgt.

4. Verfahren nach Anspruch 1, dadurch gekennzeichnet, daß das Atomverhältnis von Kobalt zu Ruthenium 1 : 0,000 5 bis 1 : 0,5 vorzugsweise 1 : 0,05 bis 1 : 0,1 beträgt.

5. Verfahren nach Anspruch 1, dadurch gekennzeichnet, daß das molare Verhältnis von Kobalt zu Jod 1 : 0,02 bis 1 : 2 vorzugsweise 1 : 0,1 bis 1 : 1 beträgt.

**Claims**

1. Process for the preparation of ethanol by the reaction of methanol with carbon monoxide and hydrogen at pressures of 200 to 600 bar and temperatures of 150° to 250 °C in the presence of 5 to 25 weight-% water related methanol and in the presence of a catalyst containing a cobalt compound, a ruthenium compound, iodine or iodide and a bidentate organic phosphorous compound characterised in that the bidentate organic phosphorus compound has the general formula :

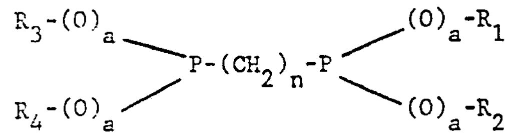

where a is 0 or 1, n is the number 1, 2, 3, 4, 5 or 6 and in the case of a being equal to 0 $R_1$ and $R_3$ are hydrogen and $R_2$ and $R_4$ are the same or different straight-chained or branched alkyl groups with 1 to 16 carbon atoms or the same or different aryl groups with 6 to 15 carbon atoms and in the case of a being equal to 1 $R_1$, $R_2$, $R_3$ and $R_4$ are the same or different and can be hydrogen, straight-chained or branched alkyl groups with 1 to 16 carbon atoms or aryl groups with 6 to 15 carbon atoms.

2. Process according to claim 1, caracterised in that in the cas of a being equal to 1 the groups $R_1$ and $R_2$ resp. $R_3$ and $R_4$ are connected to each other.

3. Process according to claims 1 and 2, characterised in that the molar ratio of cobalt to methanol is 1 : 20 to 1 : 10,000 preferably 1 : 30 to 1 : 2,000 and the molar ratio of cobalt to the phosphorous compound is 1 : 1 to 1 : 20 preferably 1 : 1 to 1 : 5.

4. Process according to claim 1, characterised in that the atomic ratio of cobalt to ruthenium is 1 : 0.000 5 to 1 : 0.5 preferably 1 : 0.05 to 1 : 0.1.

5. Process according to claim 1, characterised in that the molar ratio of cobalt to iodine is 1 : 0.02 to 1 : 2 preferably 1 : 0.1 to 1 : 1.

**Revendications**

1. Procédé de préparation de l'éthanol par réaction du méthanol avec l'oxyde de carbone et l'hydrogène sous des pressions de 200 à 600 bars et à des températures de 150 à 250 °C en présence de 5 à 25 % en poids d'eau, par rapport au méthanol, et en présence d'un catalyseur qui contient un composé du cobalt, un composé du ruthénium, de l'iode ou un iodure et un composé organique phosphoré bidentique, caractérisé en ce que le composé organique phosphoré bidentique répond à la formule générale :

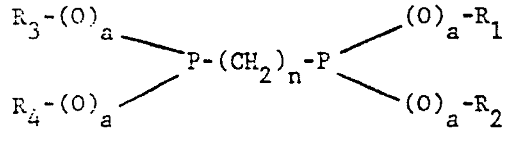

dans laquelle a est égal à 0 ou 1, n est égal à 1, 2, 3, 4, 5 ou 6 et, dans le cas où a = 0, $R_1$ et $R_3$ représentent l'hydrogène et $R_2$ et $R_4$, identiques ou différents, représentent des groupes alkyle à chaîne droite ou ramifiée contenant 1 à 16 atomes de carbone ou des groupes aryle identiques ou différents contenant 6 à 15 atomes de carbone, et, dans le cas où a = 1, $R_1$, $R_2$, $R_3$ et $R_4$, identiques ou différents, représentent l'hydrogène, des groupes alkyle à chaîne droite ou ramifiée contenant 1 à 16 atomes de carbone ou des groupes aryle contenant 6 à 15 atomes de carbone.

2. Procédé selon la revendication 1, caractérisé en ce que, lorsque a = 1, les restes $R_1$ et $R_2$ d'une part, $R_3$ et $R_4$ d'autre part, sont reliés entre eux.

3. Procédé selon les revendications 1 et 2, caractérisé en ce que le rapport molaire cobalt/méthanol est de 1 : 20 à 1 : 10 000 de préférence de 1 : 30 à 1 : 2 000 et le rapport molaire cobalt/composé phosphoré est de 1 : 1 à 1 : 20, de préférence de 1 : 1 à 1 : 5.

4. Procédé selon la revendication 1, caractérisé en ce que le rapport atomique cobalt/ruthénium est de 1 : 0,000 5 à 1 : 0,5, de préférence de 1 : 0,05 à 1 : 0,1.

5. Procédé selon la revendication 1, caractérisé en ce que le rapport molaire cobalt/iode est de 1 : 0,02 à 1 : 2, de préférence de 1 : 0,1 à 1 : 1.